# EUROPEAN PATENT APPLICATION

(11) **EP 2 366 406 A1**
(43) Date of publication of application: **21.09.2011**
(21) Application number: 10184830.7
(22) Date of filing: 24.01.2002
(51) Int. Cl.: A61K 39/395, A61K 38/17, A61P 17/06

(54) **Method for treating psoriasis**

(30) Priority: 25.01.2001 US 264219 P
(62) Divisional of application: 02760986.6
(71) Applicant: ZymoGenetics, Inc., Seattle, Washington 98102 (US)
(72) Inventor: Moore, Emma, E., Seattle, WA 98199 (US); Foley, Kevin, P., Landsdale, PA 19446 (US); Madden, Karen, L., Bellevue, WA 98004 (US); Yao, Yue, 98028, WA Kenmore (US); Presnell, Scott, R., Tacoma, WA 98407 (US)
(74) Representative: Griffin, Philippa Jane

(57) **Abstract**

The present invention describes use of an antagonist to a IL17Dβ9 (IL-17D) polypeptide in the manufacture of a medicament for the treatment of psoriatic arthritis. The antagonist can be an antibody, antibody fragment, singlechain antibody or antisense nucleotide.

SEQUENCE LISTING

<110> ZymoGenetics, Inc.

<120> Method for treating psoriasis

<130> P25809EP-D1-PCT

<140> Not yet assigned
<141> 2002-01-24

<150> US 60/264,219
<151> 2001-01-25

<150> EP02760986.6
<151> 2002-01-24

<160> 16

<170> FastSEQ for Windows Version 3.0

<210> 1
<211> 1819
<212> DNA
<213> Homo sapiens

<220>
<221> CDS
<222> (71)...(676)

<400> 1
cgggcgcggg gcgcaggcgg gctcctccgg cgcgtgcgga cgctgagcgt ggcctgtccc
60
tcaggtctgg atg ctg gta gcc ggc ttc ctg ctg gcg ctg ccg ccg agc
109
Met Leu Val Ala Gly Phe Leu Leu Ala Leu Pro Pro Ser
1               5                   10

tgg gcc gcg ggc gcc ccg agg gcg ggc agg cgc ccc gcg cgg ccg cgg
157
Trp Ala Ala Gly Ala Pro Arg Ala Gly Arg Arg Pro Ala Arg Pro Arg
    15                  20                  25

ggc tgc gcg gac cgg ccg gag gag cta ctg gag cag ctg tac ggg cgc
205
Gly Cys Ala Asp Arg Pro Glu Glu Leu Leu Glu Gln Leu Tyr Gly Arg
30                  35                  40                  45

ctg gcg gcc ggc gtg ctc agt gcc ttc cac cac acg ctg cag ctg ggg
253
Leu Ala Ala Gly Val Leu Ser Ala Phe His His Thr Leu Gln Leu Gly
                50                  55                  60

ccg cgt gag cag gcg cgc aac gcg agc tgc ccg gca ggg ggc agg ccc
301
Pro Arg Glu Gln Ala Arg Asn Ala Ser Cys Pro Ala Gly Gly Arg Pro

                 65                  70                  75

 gcc gac cgc cgc ttc cgg ccg ccc acc aac ctg cgc agc gtg tcg ccc
349
 Ala Asp Arg Arg Phe Arg Pro Pro Thr Asn Leu Arg Ser Val Ser Pro
         80                  85                  90

 tgg gcc tac aga atc tcc tac gac ccg gcg agg tac ccc agg tac ctg
397
 Trp Ala Tyr Arg Ile Ser Tyr Asp Pro Ala Arg Tyr Pro Arg Tyr Leu
     95                  100                 105

 cct gaa gcc tac tgc ctg tgc cgg ggc tgc ctg acc ggg ctg ttc ggc
445
 Pro Glu Ala Tyr Cys Leu Cys Arg Gly Cys Leu Thr Gly Leu Phe Gly
 110                 115                 120                 125

 gag gag gac gtg cgc ttc cgc agc gcc cct gtc tac atg ccc acc gtc
493
 Glu Glu Asp Val Arg Phe Arg Ser Ala Pro Val Tyr Met Pro Thr Val
                 130                 135                 140

 gtc ctg cgc cgc acc ccc gcc tgc gcc ggc ggc cgt tcc gtc tac acc
541
 Val Leu Arg Arg Thr Pro Ala Cys Ala Gly Gly Arg Ser Val Tyr Thr
             145                 150                 155

 gag gcc tac gtc acc atc ccc gtg ggc tgc acc tgc gtc ccc gag ccg
589
 Glu Ala Tyr Val Thr Ile Pro Val Gly Cys Thr Cys Val Pro Glu Pro
         160                 165                 170

 gag aag gac gca gac agc atc aac tcc agc atc gac aaa cag ggc gcc
637
 Glu Lys Asp Ala Asp Ser Ile Asn Ser Ser Ile Asp Lys Gln Gly Ala
     175                 180                 185

 aag ctc ctg ctg ggc ccc aac gac gcg ccc gct ggc ccc tgaggccggt
686
 Lys Leu Leu Leu Gly Pro Asn Asp Ala Pro Ala Gly Pro
 190                 195                 200

 cctgccccgg gaggtctccc cggcccgcat cccgaggcgc ccaagctgga gccgcctgga
746
 gggctcggtc ggcgacctct gaagagagtg caccgagcaa accaagtgcc ggagcaccag
806
 cgccgccttt ccatggagac tcgtaagcag cttcatctga cacgggaatc cctggcttgc
866
 ttttagctac aagcaagcag cgtggctgga agctgatggg aaacgacccg gcacgggcat
926
 cctgtgtgcg gcccgcatgg agggtttgga aaagttcacg gaggctccct gaggagcctc
986
 tcagatcggc tgctgcgggt gcagggcgtg actcaccgct gggtgcttgc caaagagata
1046
 gggacgcata tgctttttaa agcaatctaa aaataataat aagtatagcg actatatacc
1106

tacttttaaa atcaactgtt ttgaatagag gcagagctat tttatattat caaatgagag
1166
ctactctgtt acatttctta acatataaac atcgtttttt acttcttctg gtagaatttt
1226
ttaaagcata attggaatcc ttggataaat tttgtagctg gtacactctg gcctgggtct
1286
ctgaattcag cctgtcaccg atggctgact gatgaaatgg acacgtctca tctgacccac
1346
tcttccttcc actgaaggtc ttcacgggcc tccaggtgga ccaaagggat gcacaggcgg
1406
ctcgcatgcc ccagggccag ctaagagttc caaagatctc agatttggtt ttagtcatga
1466
atacataaac agtctcaaac tcgcacaatt ttttccccct tttgaaagcc actggggcca
1526
atttgtggtt aagaggtggt gagataagaa gtggaacgtg acatctttgc cagttgtcag
1586
aagaatccaa gcaggtattg gcttagttgt aagggcttta ggatcaggcc gaatatgagg
1646
acaaagtggg ccacgttagc atctgcagag atcaatctgg aggcttctgt ttctgcattc
1706
tgccacgaga gctaggtcct tgatcttttc tttagattga aagtctgtct ctgaacacaa
1766
ttatttgtaa aagttagaag ttctttttta aatcattaaa agaggcttgc tga
1819

<210> 2
<211> 202
<212> PRT
<213> Homo sapiens

<400> 2
Met Leu Val Ala Gly Phe Leu Leu Ala Leu Pro Pro Ser Trp Ala Ala
1               5                   10                  15
Gly Ala Pro Arg Ala Gly Arg Arg Pro Ala Arg Pro Arg Gly Cys Ala
            20                  25                  30
Asp Arg Pro Glu Glu Leu Leu Glu Gln Leu Tyr Gly Arg Leu Ala Ala
        35                  40                  45
Gly Val Leu Ser Ala Phe His His Thr Leu Gln Leu Gly Pro Arg Glu
    50                  55                  60
Gln Ala Arg Asn Ala Ser Cys Pro Ala Gly Gly Arg Pro Ala Asp Arg
65                  70                  75                  80
Arg Phe Arg Pro Pro Thr Asn Leu Arg Ser Val Ser Pro Trp Ala Tyr
                85                  90                  95
Arg Ile Ser Tyr Asp Pro Ala Arg Tyr Pro Arg Tyr Leu Pro Glu Ala
            100                 105                 110
Tyr Cys Leu Cys Arg Gly Cys Leu Thr Gly Leu Phe Gly Glu Glu Asp
        115                 120                 125
Val Arg Phe Arg Ser Ala Pro Val Tyr Met Pro Thr Val Val Leu Arg
    130                 135                 140
Arg Thr Pro Ala Cys Ala Gly Gly Arg Ser Val Tyr Thr Glu Ala Tyr
145                 150                 155                 160
Val Thr Ile Pro Val Gly Cys Thr Cys Val Pro Glu Pro Glu Lys Asp
                165                 170                 175
Ala Asp Ser Ile Asn Ser Ser Ile Asp Lys Gln Gly Ala Lys Leu Leu
            180                 185                 190
Leu Gly Pro Asn Asp Ala Pro Ala Gly Pro
        195                 200

<210> 3
<211> 187
<212> PRT
<213> Homo sapiens

<400> 3
Ala Gly Ala Pro Arg Ala Gly Arg Arg Pro Ala Arg Pro Arg Gly Cys
1               5                   10                  15
Ala Asp Arg Pro Glu Glu Leu Leu Glu Gln Leu Tyr Gly Arg Leu Ala
            20                  25                  30
Ala Gly Val Leu Ser Ala Phe His His Thr Leu Gln Leu Gly Pro Arg
        35                  40                  45
Glu Gln Ala Arg Asn Ala Ser Cys Pro Ala Gly Gly Arg Pro Ala Asp
    50                  55                  60
Arg Arg Phe Arg Pro Pro Thr Asn Leu Arg Ser Val Ser Pro Trp Ala
65                  70                  75                  80
Tyr Arg Ile Ser Tyr Asp Pro Ala Arg Tyr Pro Arg Tyr Leu Pro Glu
                85                  90                  95
Ala Tyr Cys Leu Cys Arg Gly Cys Leu Thr Gly Leu Phe Gly Glu Glu
            100                 105                 110
Asp Val Arg Phe Arg Ser Ala Pro Val Tyr Met Pro Thr Val Val Leu
        115                 120                 125
Arg Arg Thr Pro Ala Cys Ala Gly Gly Arg Ser Val Tyr Thr Glu Ala
    130                 135                 140
Tyr Val Thr Ile Pro Val Gly Cys Thr Cys Val Pro Glu Pro Glu Lys
145                 150                 155                 160
Asp Ala Asp Ser Ile Asn Ser Ser Ile Asp Lys Gln Gly Ala Lys Leu
                165                 170                 175
Leu Leu Gly Pro Asn Asp Ala Pro Ala Gly Pro
            180                 185

<210> 4
<211> 186
<212> PRT
<213> Homo sapiens

<400> 4
Gly Ala Pro Arg Ala Gly Arg Arg Pro Ala Arg Pro Arg Gly Cys Ala
1               5                   10                  15
Asp Arg Pro Glu Glu Leu Leu Glu Gln Leu Tyr Gly Arg Leu Ala Ala
            20                  25                  30
Gly Val Leu Ser Ala Phe His His Thr Leu Gln Leu Gly Pro Arg Glu
        35                  40                  45
Gln Ala Arg Asn Ala Ser Cys Pro Ala Gly Gly Arg Pro Ala Asp Arg
    50                  55                  60
Arg Phe Arg Pro Pro Thr Asn Leu Arg Ser Val Ser Pro Trp Ala Tyr
65                  70                  75                  80
Arg Ile Ser Tyr Asp Pro Ala Arg Tyr Pro Arg Tyr Leu Pro Glu Ala
                85                  90                  95
Tyr Cys Leu Cys Arg Gly Cys Leu Thr Gly Leu Phe Gly Glu Glu Asp
            100                 105                 110
Val Arg Phe Arg Ser Ala Pro Val Tyr Met Pro Thr Val Val Leu Arg
        115                 120                 125
Arg Thr Pro Ala Cys Ala Gly Gly Arg Ser Val Tyr Thr Glu Ala Tyr
    130                 135                 140
Val Thr Ile Pro Val Gly Cys Thr Cys Val Pro Glu Pro Glu Lys Asp

145                 150                 155                 160
Ala Asp Ser Ile Asn Ser Ser Ile Asp Lys Gln Gly Ala Lys Leu Leu
                165                 170                 175
Leu Gly Pro Asn Asp Ala Pro Ala Gly Pro
            180                 185

<210> 5
<211> 185
<212> PRT
<213> Homo sapiens

<400> 5
Ala Pro Arg Ala Gly Arg Arg Pro Ala Arg Pro Arg Gly Cys Ala Asp
 1               5                  10                  15
Arg Pro Glu Glu Leu Leu Glu Gln Leu Tyr Gly Arg Leu Ala Ala Gly
            20                  25                  30
Val Leu Ser Ala Phe His His Thr Leu Gln Leu Gly Pro Arg Glu Gln
        35                  40                  45
Ala Arg Asn Ala Ser Cys Pro Ala Gly Gly Arg Pro Ala Asp Arg Arg
    50                  55                  60
Phe Arg Pro Pro Thr Asn Leu Arg Ser Val Ser Pro Trp Ala Tyr Arg
65                  70                  75                  80
Ile Ser Tyr Asp Pro Ala Arg Tyr Pro Arg Tyr Leu Pro Glu Ala Tyr
                85                  90                  95
Cys Leu Cys Arg Gly Cys Leu Thr Gly Leu Phe Gly Glu Glu Asp Val
            100                 105                 110
Arg Phe Arg Ser Ala Pro Val Tyr Met Pro Thr Val Val Leu Arg Arg
        115                 120                 125
Thr Pro Ala Cys Ala Gly Gly Arg Ser Val Tyr Thr Glu Ala Tyr Val
    130                 135                 140
Thr Ile Pro Val Gly Cys Thr Cys Val Pro Glu Pro Glu Lys Asp Ala
145                 150                 155                 160
Asp Ser Ile Asn Ser Ser Ile Asp Lys Gln Gly Ala Lys Leu Leu Leu
                165                 170                 175
Gly Pro Asn Asp Ala Pro Ala Gly Pro
            180                 185

<210> 6
<211> 2361
<212> DNA
<213> Homo sapiens

<220>
<221> CDS
<222> (572)...(1202)

<400> 6
gaattcggca cgagggtcag ggaagtattc agtgctttgt tgtagagttg ttggatagag
60
gcacaggatc atttcatgtt gttgaggaga aaggagcaac agcctcctcc caccttatta
120
aaaatagaga tttaaaaaaa cctctaattt cctcgaagta cagaatctca agaggtagct
180
ctaaggagaa tccctctggg tttgagcgca ttcctcttcc aggggcccta ttcttggact
240
gctttcctta atagagaaat ctctctgagc caaaatcggc ctcccccaat tccatcctgt
300

cggccccact tttctgctcc ggagacttcc aagccagtcc ccactcctcc ttcagccagt
360
cgggcccgca cccgcgcccg gcagggccag ccctctcctc ctcctgcgtg gcgcagcaca
420
ggccctgagc gcgcgacccc aggccctggg cgccccgccg catgctcgcg gctggaagcc
480
ccagtttgcg tggcccttcg ggttattccg ctcaagagcc gccgcgtcgc cccatctcgg
540
cgcgaatctg aaagcgcttt cgggggagaa g atg ttg ggg gca ctg gtc tgg
592
                                   Met Leu Gly Ala Leu Val Trp
                                    1               5

atg ctg gta gcc ggc ttc ctg ctg gcg ctg ccg ccg agc tgg gcc gcg
640
Met Leu Val Ala Gly Phe Leu Leu Ala Leu Pro Pro Ser Trp Ala Ala
        10                  15                  20

ggc gcc ccg agg gcg ggc agg cgc ccc gcg cgg ccg cgg ggc tgc gcg
688
Gly Ala Pro Arg Ala Gly Arg Arg Pro Ala Arg Pro Arg Gly Cys Ala
    25                  30                  35

gac cgg ccg gag gag cta ctg gag cag ctg tac ggg cgc ctg gcg gcc
736
Asp Arg Pro Glu Glu Leu Leu Glu Gln Leu Tyr Gly Arg Leu Ala Ala
 40                  45                  50                  55

ggc gtg ctc agt gcc ttc cac cac acg ctg cag ctg ggg ccg cgt gag
784
Gly Val Leu Ser Ala Phe His His Thr Leu Gln Leu Gly Pro Arg Glu
                60                  65                  70

cag gcg cgc aac gcg agc tgc ccg gca ggg ggc agg ccc gcc gac cgc
832
Gln Ala Arg Asn Ala Ser Cys Pro Ala Gly Gly Arg Pro Ala Asp Arg
            75                  80                  85

cgc ttc cgg ccg ccc acc aac ctg cgc agc gtg tcg ccc tgg gcc tac
880
Arg Phe Arg Pro Pro Thr Asn Leu Arg Ser Val Ser Pro Trp Ala Tyr
        90                  95                  100

aga atc tcc tac gac ccg gcg agg tac ccc agg tac ctg cct gaa gcc
928
Arg Ile Ser Tyr Asp Pro Ala Arg Tyr Pro Arg Tyr Leu Pro Glu Ala
    105                 110                 115

tac tgc ctg tgc cgg ggc tgc ctg acc ggg ctg ttc ggc gag gag gac
976
Tyr Cys Leu Cys Arg Gly Cys Leu Thr Gly Leu Phe Gly Glu Glu Asp
120                 125                 130                 135

gtg cgc ttc cgc agc gcc cct gtc tac atg ccc acc gtc gtc ctg cgc
1024
Val Arg Phe Arg Ser Ala Pro Val Tyr Met Pro Thr Val Val Leu Arg
                140                 145                 150

cgc acc ccc gcc tgc gcc ggc ggc cgt tcc gtc tac acc gag gcc tac
1072
Arg Thr Pro Ala Cys Ala Gly Gly Arg Ser Val Tyr Thr Glu Ala Tyr
            155                 160                 165

gtc acc atc ccc gtg ggc tgc acc tgc gtc ccc gag ccg gag aag gac
1120
Val Thr Ile Pro Val Gly Cys Thr Cys Val Pro Glu Pro Glu Lys Asp
        170                 175                 180

gca gac agc atc aac tcc agc atc gac aaa cag ggc gcc aag ctc ctg
1168
Ala Asp Ser Ile Asn Ser Ser Ile Asp Lys Gln Gly Ala Lys Leu Leu
    185                 190                 195

ctg ggc ccc aac gac gcg ccc gct ggc ccc tga g gccggtcctg
1212
Leu Gly Pro Asn Asp Ala Pro Ala Gly Pro  *
200                 205

ccccgggagg tctccccggc ccgcatcccg aggcgcccaa gctggagccg cctggagggc
1272
tcggtcggcg acctctgaag agagtgcacc gagcaaacca agtgccggag caccagcgcc
1332
gcctttccat ggagactcgt aagcagcttc atctgacacg ggaatccctg gcttgctttt
1392
agctacaagc aagcagcgtg gctggaagct gatgggaaac gacccggcac gggcatcctg
1452
tgtgcggccc gcatggaggg tttggaaaag ttcacggagg ctccctgagg agcctctcag
1512
atcggctgct gcgggtgcag ggcgtgactc accgctgggt gcttgccaaa gagataggga
1572
cgcatatgct ttttaaagca atctaaaaat aataataagt atagcgacta tatacctact
1632
tttaaaatca actgttttga atagaggcag agctatttta tattatcaaa tgagagctac
1692
tctgttacat ttcttaacat ataaacatcg ttttttactt cttctggtag aattttttaa
1752
agcataattg gaatccttgg ataaattttg tagctggtac actctggcct gggtctctga
1812
attcagcctg tcaccgatgg ctgactgatg aaatggacac gtctcatctg acccactctt
1872
ccttccactg aaggtcttca cgggcctcca ggtggaccaa agggatgcac aggcggctcg
1932
catgccccag ggccagctaa gagttccaaa gatctcagat ttggttttag tcatgaatac
1992
ataaacagtc tcaaactcgc acaatttttt cccccttttg aaagccactg gggccaattt
2052
gtggttaaga ggtggtgaga taagaagtgg aacgtgacat ctttgccagt tgtcagaaga
2112
atccaagcag gtattggctt agttgtaagg gctttaggat caggccgaat atgaggacaa
2172
agtgggccac gttagcatct gcagagatca atctggaggc ttctgtttct gcattctgcc
2232
acgagagcta ggtccttgat cttttcttta gattgaaagt ctgtctctga acacaattat
2292

ttgtaaaagt tagaagttct tttttaaatc attaaaagag gcttgctgaa aaaaaaaaaa
2352
aaaaaaaaa
2361

<210> 7
<211> 209
<212> PRT
<213> Homo sapiens

<400> 7
Met Leu Gly Ala Leu Val Trp Met Leu Val Ala Gly Phe Leu Leu Ala
1               5                   10                  15
Leu Pro Pro Ser Trp Ala Ala Gly Ala Pro Arg Ala Gly Arg Arg Pro
            20                  25                  30
Ala Arg Pro Arg Gly Cys Ala Asp Arg Pro Glu Glu Leu Leu Glu Gln
        35                  40                  45
Leu Tyr Gly Arg Leu Ala Ala Gly Val Leu Ser Ala Phe His His Thr
    50                  55                  60
Leu Gln Leu Gly Pro Arg Glu Gln Ala Arg Asn Ala Ser Cys Pro Ala
65                  70                  75                  80
Gly Gly Arg Pro Ala Asp Arg Arg Phe Arg Pro Pro Thr Asn Leu Arg
                85                  90                  95
Ser Val Ser Pro Trp Ala Tyr Arg Ile Ser Tyr Asp Pro Ala Arg Tyr
            100                 105                 110
Pro Arg Tyr Leu Pro Glu Ala Tyr Cys Leu Cys Arg Gly Cys Leu Thr
        115                 120                 125
Gly Leu Phe Gly Glu Glu Asp Val Arg Phe Arg Ser Ala Pro Val Tyr
    130                 135                 140
Met Pro Thr Val Val Leu Arg Arg Thr Pro Ala Cys Ala Gly Gly Arg
145                 150                 155                 160
Ser Val Tyr Thr Glu Ala Tyr Val Thr Ile Pro Val Gly Cys Thr Cys
                165                 170                 175
Val Pro Glu Pro Glu Lys Asp Ala Asp Ser Ile Asn Ser Ser Ile Asp
            180                 185                 190
Lys Gln Gly Ala Lys Leu Leu Leu Gly Pro Asn Asp Ala Pro Ala Gly
        195                 200                 205
Pro


<210> 8
<211> 187
<212> PRT
<213> Homo sapiens

<400> 8
Ala Gly Ala Pro Arg Ala Gly Arg Arg Pro Ala Arg Pro Arg Gly Cys
1               5                   10                  15
Ala Asp Arg Pro Glu Glu Leu Leu Glu Gln Leu Tyr Gly Arg Leu Ala
            20                  25                  30
Ala Gly Val Leu Ser Ala Phe His His Thr Leu Gln Leu Gly Pro Arg
        35                  40                  45
Glu Gln Ala Arg Asn Ala Ser Cys Pro Ala Gly Gly Arg Pro Ala Asp
    50                  55                  60
Arg Arg Phe Arg Pro Pro Thr Asn Leu Arg Ser Val Ser Pro Trp Ala
65                  70                  75                  80
Tyr Arg Ile Ser Tyr Asp Pro Ala Arg Tyr Pro Arg Tyr Leu Pro Glu

                 85                  90                  95
Ala Tyr Cys Leu Cys Arg Gly Cys Leu Thr Gly Leu Phe Gly Glu Glu
            100                 105                 110
Asp Val Arg Phe Arg Ser Ala Pro Val Tyr Met Pro Thr Val Val Leu
        115                 120                 125
Arg Arg Thr Pro Ala Cys Ala Gly Gly Arg Ser Val Tyr Thr Glu Ala
    130                 135                 140
Tyr Val Thr Ile Pro Val Gly Cys Thr Cys Val Pro Glu Pro Glu Lys
145                 150                 155                 160
Asp Ala Asp Ser Ile Asn Ser Ser Ile Asp Lys Gln Gly Ala Lys Leu
                165                 170                 175
Leu Leu Gly Pro Asn Asp Ala Pro Ala Gly Pro
            180                 185

<210> 9
<211> 1221
<212> DNA
<213> Mus musculis

<220>
<221> CDS
<222> (79)...(693)

<400> 9
gggtgtcgcc cttatttact tcgcagaaga gccttcagcc cccctcctaa caagtctgga
60
aagcatcacg gcgacgcg atg ttg ggg aca ctg gtc tgg atg ctc gcg gtc
111
                    Met Leu Gly Thr Leu Val Trp Met Leu Ala Val
                     1               5                  10

ggc ttc ctg ctg gca ctg gcg ccg ggc cgc gcg gcg ggc gcg ctg agg
159
Gly Phe Leu Leu Ala Leu Ala Pro Gly Arg Ala Ala Gly Ala Leu Arg
            15                  20                  25

acc ggg agg cgc ccg gcg cgg ccg cgg gac tgc gcg gac cgg ccg gag
207
Thr Gly Arg Arg Pro Ala Arg Pro Arg Asp Cys Ala Asp Arg Pro Glu
        30                  35                  40

gag ctc ctg gag cag ctg tac ggg cgg ctg gcg gcc ggc gtg ctc agc
255
Glu Leu Leu Glu Gln Leu Tyr Gly Arg Leu Ala Ala Gly Val Leu Ser
    45                  50                  55

gcc ttc cac cac acg ctg cag ctc ggg ccg cgc gag cag gcg cgc aat
303
Ala Phe His His Thr Leu Gln Leu Gly Pro Arg Glu Gln Ala Arg Asn
 60                  65                  70                  75

gcc agc tgc ccg gcc ggg ggc agg gcc gcc gac cgc cgc ttc cgg cca
351
Ala Ser Cys Pro Ala Gly Gly Arg Ala Ala Asp Arg Arg Phe Arg Pro
                80                  85                  90

ccc acc aac ctg cgc agc gtg tcg ccc tgg gcg tac agg att tcc tac
399
Pro Thr Asn Leu Arg Ser Val Ser Pro Trp Ala Tyr Arg Ile Ser Tyr
            95                  100                 105

gac cct gct cgc ttt ccg agg tac ctg ccc gaa gcc tac tgc ctg tgc
447
Asp Pro Ala Arg Phe Pro Arg Tyr Leu Pro Glu Ala Tyr Cys Leu Cys
        110                 115                 120

cga ggc tgc ctg acc ggg ctc tac ggg gag gag gac ttc cgc ttt cgc
495
Arg Gly Cys Leu Thr Gly Leu Tyr Gly Glu Glu Asp Phe Arg Phe Arg
    125                 130                 135

agc aca ccc gtc ttc tct cca gcc gtg gtg ctg cgg cgc aca gcg gcc
543
Ser Thr Pro Val Phe Ser Pro Ala Val Val Leu Arg Arg Thr Ala Ala
140                 145                 150                 155

tgc gcg ggc ggc cgc tct gtg tac gcc gaa cac tac atc acc atc ccg
591
Cys Ala Gly Gly Arg Ser Val Tyr Ala Glu His Tyr Ile Thr Ile Pro
                160                 165                 170

gtg ggc tgc acc tgc gtg ccc gag ccg gac aag tcc gcg gac agt gcg
639
Val Gly Cys Thr Cys Val Pro Glu Pro Asp Lys Ser Ala Asp Ser Ala
            175                 180                 185

aac tcc agc atg gac aag ctg ctg ctg ggg ccc gcc gac agg cct gcg
687
Asn Ser Ser Met Asp Lys Leu Leu Leu Gly Pro Ala Asp Arg Pro Ala
        190                 195                 200

ggg cgc tgatgccggg gactgcccgc catggcccag cttcctgcat gcatcaggtc
743
Gly Arg
    205

ccctggccct gacaaaaccc accccatgat ccctggccgc tgcctaattt ttccaaaagg
803
acagctacat aagctttaaa tatatttttc aaagtagaca ctacatatct acaactattt
863
tgaatagtgg cagaaactat tttcatatta gtaatttaga gcaagcatgt tgtttttaaa
923
cttctttgat atacaagcac atcacacaca tcccgttttc ctctagtagg attcttgagt
983
gcataattgt agtgctcaga tgaacttcct tctgctgcac tgtgccctgt ccctgagtct
1043
ctcctgtggc ccaagcttac taaggtgata atgagtgctc cggatctggg cacctaaggt
1103
ctccaggtcc ctggagaggg agggatgtgg gggggctaga accaagcgcc cctttgttct
1163
ttagcttatg gatggtctta actttataaa gattaaagtt tttggtgtta ttctttca
1221

<210> 10
<211> 205
<212> PRT
<213> Mus musculis

<400> 10
Met Leu Gly Thr Leu Val Trp Met Leu Ala Val Gly Phe Leu Leu Ala
1               5                   10                  15
Leu Ala Pro Gly Arg Ala Ala Gly Ala Leu Arg Thr Gly Arg Arg Pro
            20                  25                  30
Ala Arg Pro Arg Asp Cys Ala Asp Arg Pro Glu Glu Leu Leu Glu Gln
        35                  40                  45
Leu Tyr Gly Arg Leu Ala Ala Gly Val Leu Ser Ala Phe His His Thr
    50                  55                  60
Leu Gln Leu Gly Pro Arg Glu Gln Ala Arg Asn Ala Ser Cys Pro Ala
65                  70                  75                  80
Gly Gly Arg Ala Ala Asp Arg Arg Phe Arg Pro Pro Thr Asn Leu Arg
                85                  90                  95
Ser Val Ser Pro Trp Ala Tyr Arg Ile Ser Tyr Asp Pro Ala Arg Phe
            100                 105                 110
Pro Arg Tyr Leu Pro Glu Ala Tyr Cys Leu Cys Arg Gly Cys Leu Thr
        115                 120                 125
Gly Leu Tyr Gly Glu Glu Asp Phe Arg Phe Arg Ser Thr Pro Val Phe
    130                 135                 140
Ser Pro Ala Val Val Leu Arg Arg Thr Ala Ala Cys Ala Gly Gly Arg
145                 150                 155                 160
Ser Val Tyr Ala Glu His Tyr Ile Thr Ile Pro Val Gly Cys Thr Cys
                165                 170                 175
Val Pro Glu Pro Asp Lys Ser Ala Asp Ser Ala Asn Ser Ser Met Asp
            180                 185                 190
Lys Leu Leu Leu Gly Pro Ala Asp Arg Pro Ala Gly Arg
        195                 200                 205

<210> 11
<211> 183
<212> PRT
<213> Mus musculis

<400> 11
Ala Gly Ala Leu Arg Thr Gly Arg Arg Pro Ala Arg Pro Arg Asp Cys
1               5                   10                  15
Ala Asp Arg Pro Glu Glu Leu Leu Glu Gln Leu Tyr Gly Arg Leu Ala
            20                  25                  30
Ala Gly Val Leu Ser Ala Phe His His Thr Leu Gln Leu Gly Pro Arg
        35                  40                  45
Glu Gln Ala Arg Asn Ala Ser Cys Pro Ala Gly Gly Arg Ala Ala Asp
    50                  55                  60
Arg Arg Phe Arg Pro Pro Thr Asn Leu Arg Ser Val Ser Pro Trp Ala
65                  70                  75                  80
Tyr Arg Ile Ser Tyr Asp Pro Ala Arg Phe Pro Arg Tyr Leu Pro Glu
                85                  90                  95
Ala Tyr Cys Leu Cys Arg Gly Cys Leu Thr Gly Leu Tyr Gly Glu Glu
            100                 105                 110
Asp Phe Arg Phe Arg Ser Thr Pro Val Phe Ser Pro Ala Val Val Leu
        115                 120                 125
Arg Arg Thr Ala Ala Cys Ala Gly Gly Arg Ser Val Tyr Ala Glu His
    130                 135                 140

Tyr Ile Thr Ile Pro Val Gly Cys Thr Cys Val Pro Glu Pro Asp Lys
145                 150                 155                 160
Ser Ala Asp Ser Ala Asn Ser Ser Met Asp Lys Leu Leu Leu Gly Pro
                165                 170                 175
Ala Asp Arg Pro Ala Gly Arg
            180

<210> 12
<211> 3016
<212> DNA
<213> Homo sapiens

<220>
<221> CDS
<222> (12)...(2864)

<400> 12
tggattacac a atg aca tgg aga atg gga ccc cgt ttc act atg ctg ttg
50
             Met Thr Trp Arg Met Gly Pro Arg Phe Thr Met Leu Leu
              1               5                  10

gcc atg tgg cta gtg tgt gga tca gaa ccc cac ccc cat gcc act att
98
Ala Met Trp Leu Val Cys Gly Ser Glu Pro His Pro His Ala Thr Ile
     15                  20                  25

aga ggc agc cac gga gga cgg aaa gtg cct ttg gtt tct ccg gac agc
146
Arg Gly Ser His Gly Gly Arg Lys Val Pro Leu Val Ser Pro Asp Ser
 30                  35                  40                  45

agt agg cca gct cgg ttt ctg agg cac act ggg agg tct cgc gga att
194
Ser Arg Pro Ala Arg Phe Leu Arg His Thr Gly Arg Ser Arg Gly Ile
                 50                  55                  60

gag aga tcc act ctg gag gaa cca aac ctt cag cct ctc cag aga agg
242
Glu Arg Ser Thr Leu Glu Glu Pro Asn Leu Gln Pro Leu Gln Arg Arg
             65                  70                  75

agg agt gtg ccc gtg ttg aga cta gct cgc cca aca gag ccg cca gcc
290
Arg Ser Val Pro Val Leu Arg Leu Ala Arg Pro Thr Glu Pro Pro Ala
         80                  85                  90

cgc tcg gac atc aat ggg gcc gcc gtg aga cct gag caa aga cca gca
338
Arg Ser Asp Ile Asn Gly Ala Ala Val Arg Pro Glu Gln Arg Pro Ala
     95                 100                 105

gcc agg ggc tct ccg cgt gag atg atc aga gat gag ggg tcc tca gct
386
Ala Arg Gly Ser Pro Arg Glu Met Ile Arg Asp Glu Gly Ser Ser Ala
110                 115                 120                 125

cgg tca aga atg ttg cgt ttc cct tcg ggg tcc agc tct ccc aac atc
434
Arg Ser Arg Met Leu Arg Phe Pro Ser Gly Ser Ser Ser Pro Asn Ile
                130                 135                 140

ctt gcc agc ttt gca ggg aag aac aga gta tgg gtc atc tca gcc cct
482
Leu Ala Ser Phe Ala Gly Lys Asn Arg Val Trp Val Ile Ser Ala Pro
            145                 150                 155

cat gcc tcg gaa ggc tac tac cgc ctc atg atg agc ctg ctg aag gac
530
His Ala Ser Glu Gly Tyr Tyr Arg Leu Met Met Ser Leu Leu Lys Asp
        160                 165                 170

gat gtg tac tgt gag ctg gcg gag agg cac atc caa cag att gtg ctc
578
Asp Val Tyr Cys Glu Leu Ala Glu Arg His Ile Gln Gln Ile Val Leu
    175                 180                 185

ttc cac cag gca ggt gag gaa gga ggc aag gtg aga agg atc acc agc
626
Phe His Gln Ala Gly Glu Glu Gly Gly Lys Val Arg Arg Ile Thr Ser
190                 195                 200                 205

gag ggc cag atc ctg gag cag ccc ctg gac cct agc ctc atc cct aag
674
Glu Gly Gln Ile Leu Glu Gln Pro Leu Asp Pro Ser Leu Ile Pro Lys
                210                 215                 220

ctg atg agc ttc ctg aag ctg gag aag ggc aag ttt ggc atg gtg ctg
722
Leu Met Ser Phe Leu Lys Leu Glu Lys Gly Lys Phe Gly Met Val Leu
            225                 230                 235

ctg aag aag acg ctg cag gtg gag gag cgc tat cca tat ccc gtt agg
770
Leu Lys Lys Thr Leu Gln Val Glu Glu Arg Tyr Pro Tyr Pro Val Arg
        240                 245                 250

ctg gaa gcc atg tac gag gtc atc gac caa ggc ccc atc cgt agg atc
818
Leu Glu Ala Met Tyr Glu Val Ile Asp Gln Gly Pro Ile Arg Arg Ile
    255                 260                 265

gag aag atc agg cag aag ggc ttt gtc cag aaa tgt aag gcc tct ggt
866
Glu Lys Ile Arg Gln Lys Gly Phe Val Gln Lys Cys Lys Ala Ser Gly
270                 275                 280                 285

gta gag ggc cag gtg gtg gcg gag ggg aat gac ggt gga ggg gga gca
914
Val Glu Gly Gln Val Val Ala Glu Gly Asn Asp Gly Gly Gly Gly Ala
                290                 295                 300

gga agg cca agc ctg ggc agc gag aag aag aaa gag gac cca agg aga
962

Gly Arg Pro Ser Leu Gly Ser Glu Lys Lys Lys Glu Asp Pro Arg Arg
            305                 310                 315

gca caa gtc cca cca acc aga gag agt cgg gtg aag gtc ctg aga aaa
1010
Ala Gln Val Pro Pro Thr Arg Glu Ser Arg Val Lys Val Leu Arg Lys
        320                 325                 330

ctg gcc gcc act gca cca gct ttg ccc caa cct ccc tca acc ccc aga
1058
Leu Ala Ala Thr Ala Pro Ala Leu Pro Gln Pro Pro Ser Thr Pro Arg
    335                 340                 345

gcc acc acc ctt cct cct gcc cca gcc aca aca gtg act cgg tcc acg
1106
Ala Thr Thr Leu Pro Pro Ala Pro Ala Thr Thr Val Thr Arg Ser Thr
350                 355                 360                 365

tcc cgg gcg gta aca gtt gct gca aga cct atg acc acc act gcc ttt
1154
Ser Arg Ala Val Thr Val Ala Ala Arg Pro Met Thr Thr Thr Ala Phe
                370                 375                 380

ccc acc acg cag agg ccc tgg acc ccc tca ccc tcc cac agg ccc cct
1202
Pro Thr Thr Gln Arg Pro Trp Thr Pro Ser Pro Ser His Arg Pro Pro
            385                 390                 395

aca acc act gag gtg atc act gcc agg aga ccc tca gtt tca gag aat
1250
Thr Thr Thr Glu Val Ile Thr Ala Arg Arg Pro Ser Val Ser Glu Asn
        400                 405                 410

ctt tac cct cca tcc cgg aag gat cag cac agg gag agg cca cag aca
1298
Leu Tyr Pro Pro Ser Arg Lys Asp Gln His Arg Glu Arg Pro Gln Thr
    415                 420                 425

acc agg agg ccc agc aag gcc acc agc ttg gag agc ttc aca aat gcc
1346
Thr Arg Arg Pro Ser Lys Ala Thr Ser Leu Glu Ser Phe Thr Asn Ala
430                 435                 440                 445

cct ccc acc acc atc tca gaa ccc agc aca agg gct gct ggc cca ggc
1394
Pro Pro Thr Thr Ile Ser Glu Pro Ser Thr Arg Ala Ala Gly Pro Gly
                450                 455                 460

cgt ttc cgg gac aac cgc atg gac agg cgg gaa cat ggc cac cga gac
1442
Arg Phe Arg Asp Asn Arg Met Asp Arg Arg Glu His Gly His Arg Asp
            465                 470                 475

cca aat gtg gtg cca ggt cct ccc aag cca gca aag gag aaa cct ccc
1490
Pro Asn Val Val Pro Gly Pro Pro Lys Pro Ala Lys Glu Lys Pro Pro
        480                 485                 490

aaa aag aag gcc cag gac aaa att ctt agt aat gag tat gag gag aag
1538
Lys Lys Lys Ala Gln Asp Lys Ile Leu Ser Asn Glu Tyr Glu Glu Lys
    495                 500                 505

tat gac ctc agc cgg cct act gcc tct cag ctg gag gac gag ctg cag
1586
Tyr Asp Leu Ser Arg Pro Thr Ala Ser Gln Leu Glu Asp Glu Leu Gln
510                 515                 520                 525

gtg ggg aat gtt ccc ctt aaa aaa gca aag gag tct aaa aag cat gaa
1634
Val Gly Asn Val Pro Leu Lys Lys Ala Lys Glu Ser Lys Lys His Glu
                530                 535                 540

aag ctt gag aaa cca gag aag gag aag aaa aaa aag atg aag aat gag
1682
Lys Leu Glu Lys Pro Glu Lys Glu Lys Lys Lys Lys Met Lys Asn Glu
            545                 550                 555

aac gca gac aag tta ctt aag agt gaa aag caa atg aag aag tct gag
1730
Asn Ala Asp Lys Leu Leu Lys Ser Glu Lys Gln Met Lys Lys Ser Glu
        560                 565                 570

aaa aag agc aag caa gag aaa gag aag agc aag aag aaa aaa gga ggt
1778
Lys Lys Ser Lys Gln Glu Lys Glu Lys Ser Lys Lys Lys Lys Gly Gly
    575                 580                 585

aaa aca gaa cag gat ggc tat cag aaa ccc acc aac aaa cac ttc acg
1826
Lys Thr Glu Gln Asp Gly Tyr Gln Lys Pro Thr Asn Lys His Phe Thr
590                 595                 600                 605

cag agt ccc aag aag tca gtg gcc gac ctg ctg ggg tcc ttt gaa ggc
1874
Gln Ser Pro Lys Lys Ser Val Ala Asp Leu Leu Gly Ser Phe Glu Gly
                610                 615                 620

aaa cga aga ctc ctt ctg atc act gct ccc aag gct gag aac aat atg
1922
Lys Arg Arg Leu Leu Leu Ile Thr Ala Pro Lys Ala Glu Asn Asn Met
            625                 630                 635

tat gtg caa caa cgt gat gaa tat ctg gaa agt ttc tgc aag atg gct
1970
Tyr Val Gln Gln Arg Asp Glu Tyr Leu Glu Ser Phe Cys Lys Met Ala
        640                 645                 650

acc agg aaa atc tct gtg atc acc atc ttc ggc cct gtc aac aac agc
2018
Thr Arg Lys Ile Ser Val Ile Thr Ile Phe Gly Pro Val Asn Asn Ser
    655                 660                 665

acc atg aaa atc gac cac ttt cag cta gat aat gag aag ccc atg cga
2066
Thr Met Lys Ile Asp His Phe Gln Leu Asp Asn Glu Lys Pro Met Arg
670                 675                 680                 685

gtg gtg gat gat gaa gac ttg gta gac cag cgt ctc atc agc gag ctg
2114
Val Val Asp Asp Glu Asp Leu Val Asp Gln Arg Leu Ile Ser Glu Leu
                690                 695                 700

agg aaa gag tac gga atg acc tac aat gac ttc ttc atg gtg cta aca
2162
Arg Lys Glu Tyr Gly Met Thr Tyr Asn Asp Phe Phe Met Val Leu Thr
            705                 710                 715

gat gtg gat ctg aga gtc aag caa tac tat gag gta cca ata aca atg
2210
Asp Val Asp Leu Arg Val Lys Gln Tyr Tyr Glu Val Pro Ile Thr Met
        720                 725                 730

aag tct gtg ttt gat ctg atc gat act ttc cag tcc cga atc aaa gat
2258
Lys Ser Val Phe Asp Leu Ile Asp Thr Phe Gln Ser Arg Ile Lys Asp
    735                 740                 745

atg gag aag cag aag aag gag ggc att gtt tgc aaa gag gac aaa aag
2306
Met Glu Lys Gln Lys Lys Glu Gly Ile Val Cys Lys Glu Asp Lys Lys
750                 755                 760                 765

cag tcc ctg gag aac ttc cta tcc agg ttc cgg tgg agg agg agg ttg
2354
Gln Ser Leu Glu Asn Phe Leu Ser Arg Phe Arg Trp Arg Arg Arg Leu
                770                 775                 780

ctg gtg atc tct gct cct aac gat gaa gac tgg gcc tat tca cag cag
2402
Leu Val Ile Ser Ala Pro Asn Asp Glu Asp Trp Ala Tyr Ser Gln Gln
            785                 790                 795

ctc tct gcc ctc agt ggt cag gcg tgc aat ttt ggt ctg cgc cac ata
2450
Leu Ser Ala Leu Ser Gly Gln Ala Cys Asn Phe Gly Leu Arg His Ile
        800                 805                 810

acc att ctg aag ctt tta ggc gtt gga gag gaa gtt ggg gga gtg tta
2498
Thr Ile Leu Lys Leu Leu Gly Val Gly Glu Glu Val Gly Gly Val Leu
    815                 820                 825

gaa ctg ttc cca att aat ggg agc tct gtt gtt gag cga gaa gac gta
2546
Glu Leu Phe Pro Ile Asn Gly Ser Ser Val Val Glu Arg Glu Asp Val
830                 835                 840                 845

cca gcc cat ttg gtg aaa gac att cgt aac tat ttt caa gtg agc ccg
2594

Pro Ala His Leu Val Lys Asp Ile Arg Asn Tyr Phe Gln Val Ser Pro
                850                 855                 860

gag tac ttc tcc atg ctt cta gtc gga aaa gac gga aat gtc aaa tcc
2642
Glu Tyr Phe Ser Met Leu Leu Val Gly Lys Asp Gly Asn Val Lys Ser
            865                 870                 875

tgg tat cct tcc cca atg tgg tcc atg gtg att gtg tac gat tta att
2690
Trp Tyr Pro Ser Pro Met Trp Ser Met Val Ile Val Tyr Asp Leu Ile
        880                 885                 890

gat tcg atg caa ctt cgg aga cag gaa atg gcg att cag cag tca ctg
2738
Asp Ser Met Gln Leu Arg Arg Gln Glu Met Ala Ile Gln Gln Ser Leu
    895                 900                 905

ggg atg cgc tgc cca gaa gat gag tat gca ggc tat ggt tac cat agt
2786
Gly Met Arg Cys Pro Glu Asp Glu Tyr Ala Gly Tyr Gly Tyr His Ser
910                 915                 920                 925

tac cac caa gga tac cag gat ggt tac cag gat gac tac cgt cat cat
2834
Tyr His Gln Gly Tyr Gln Asp Gly Tyr Gln Asp Asp Tyr Arg His His
                930                 935                 940

gag agt tat cac cat gga tac cct tac tga gcagaaatat gtaaccttag
2884
Glu Ser Tyr His His Gly Tyr Pro Tyr  *
            945                 950

actcagccag tttcctctgc agctgctaaa actacatgtg gccagctcca ttcttccaca
2944
ctgcgtacta catttcctgc ctttttcttt cagtgttttt ctaagactaa ataaatagca
3004
aactttcacc ta
3016

<210> 13
<211> 950
<212> PRT
<213> Homo sapiens

<400> 13
Met Thr Trp Arg Met Gly Pro Arg Phe Thr Met Leu Leu Ala Met Trp
 1               5                  10                  15
Leu Val Cys Gly Ser Glu Pro His Pro His Ala Thr Ile Arg Gly Ser
            20                  25                  30
His Gly Gly Arg Lys Val Pro Leu Val Ser Pro Asp Ser Ser Arg Pro
        35                  40                  45
Ala Arg Phe Leu Arg His Thr Gly Arg Ser Arg Gly Ile Glu Arg Ser
    50                  55                  60
Thr Leu Glu Glu Pro Asn Leu Gln Pro Leu Gln Arg Arg Arg Ser Val
65                  70                  75                  80
Pro Val Leu Arg Leu Ala Arg Pro Thr Glu Pro Pro Ala Arg Ser Asp

85                  90                  95
Ile Asn Gly Ala Ala Val Arg Pro Glu Gln Arg Pro Ala Ala Arg Gly
            100                 105                 110
Ser Pro Arg Glu Met Ile Arg Asp Glu Gly Ser Ser Ala Arg Ser Arg
        115                 120                 125
Met Leu Arg Phe Pro Ser Gly Ser Ser Ser Pro Asn Ile Leu Ala Ser
    130                 135                 140
Phe Ala Gly Lys Asn Arg Val Trp Val Ile Ser Ala Pro His Ala Ser
145                 150                 155                 160
Glu Gly Tyr Tyr Arg Leu Met Met Ser Leu Leu Lys Asp Asp Val Tyr
                165                 170                 175
Cys Glu Leu Ala Glu Arg His Ile Gln Gln Ile Val Leu Phe His Gln
            180                 185                 190
Ala Gly Glu Glu Gly Gly Lys Val Arg Arg Ile Thr Ser Glu Gly Gln
        195                 200                 205
Ile Leu Glu Gln Pro Leu Asp Pro Ser Leu Ile Pro Lys Leu Met Ser
    210                 215                 220
Phe Leu Lys Leu Glu Lys Gly Lys Phe Gly Met Val Leu Leu Lys Lys
225                 230                 235                 240
Thr Leu Gln Val Glu Glu Arg Tyr Pro Tyr Pro Val Arg Leu Glu Ala
                245                 250                 255
Met Tyr Glu Val Ile Asp Gln Gly Pro Ile Arg Arg Ile Glu Lys Ile
            260                 265                 270
Arg Gln Lys Gly Phe Val Gln Lys Cys Lys Ala Ser Gly Val Glu Gly
        275                 280                 285
Gln Val Val Ala Glu Gly Asn Asp Gly Gly Gly Gly Ala Gly Arg Pro
    290                 295                 300
Ser Leu Gly Ser Glu Lys Lys Lys Glu Asp Pro Arg Arg Ala Gln Val
305                 310                 315                 320
Pro Pro Thr Arg Glu Ser Arg Val Lys Val Leu Arg Lys Leu Ala Ala
                325                 330                 335
Thr Ala Pro Ala Leu Pro Gln Pro Pro Ser Thr Pro Arg Ala Thr Thr
            340                 345                 350
Leu Pro Pro Ala Pro Ala Thr Thr Val Thr Arg Ser Thr Ser Arg Ala
        355                 360                 365
Val Thr Val Ala Ala Arg Pro Met Thr Thr Thr Ala Phe Pro Thr Thr
    370                 375                 380
Gln Arg Pro Trp Thr Pro Ser Pro Ser His Arg Pro Pro Thr Thr Thr
385                 390                 395                 400
Glu Val Ile Thr Ala Arg Arg Pro Ser Val Ser Glu Asn Leu Tyr Pro
                405                 410                 415
Pro Ser Arg Lys Asp Gln His Arg Glu Arg Pro Gln Thr Thr Arg Arg
            420                 425                 430
Pro Ser Lys Ala Thr Ser Leu Glu Ser Phe Thr Asn Ala Pro Pro Thr
        435                 440                 445
Thr Ile Ser Glu Pro Ser Thr Arg Ala Ala Gly Pro Gly Arg Phe Arg
    450                 455                 460
Asp Asn Arg Met Asp Arg Arg Glu His Gly His Arg Asp Pro Asn Val
465                 470                 475                 480
Val Pro Gly Pro Pro Lys Pro Ala Lys Glu Lys Pro Pro Lys Lys Lys
                485                 490                 495
Ala Gln Asp Lys Ile Leu Ser Asn Glu Tyr Glu Glu Lys Tyr Asp Leu
            500                 505                 510
Ser Arg Pro Thr Ala Ser Gln Leu Glu Asp Glu Leu Gln Val Gly Asn
        515                 520                 525
Val Pro Leu Lys Lys Ala Lys Glu Ser Lys Lys His Glu Lys Leu Glu
    530                 535                 540

Lys Pro Glu Lys Glu Lys Lys Lys Lys Met Lys Asn Glu Asn Ala Asp
545                 550                 555                 560
Lys Leu Leu Lys Ser Glu Lys Gln Met Lys Lys Ser Glu Lys Lys Ser
                565                 570                 575
Lys Gln Glu Lys Glu Lys Ser Lys Lys Lys Lys Gly Gly Lys Thr Glu
            580                 585                 590
Gln Asp Gly Tyr Gln Lys Pro Thr Asn Lys His Phe Thr Gln Ser Pro
        595                 600                 605
Lys Lys Ser Val Ala Asp Leu Leu Gly Ser Phe Glu Gly Lys Arg Arg
    610                 615                 620
Leu Leu Leu Ile Thr Ala Pro Lys Ala Glu Asn Asn Met Tyr Val Gln
625                 630                 635                 640
Gln Arg Asp Glu Tyr Leu Glu Ser Phe Cys Lys Met Ala Thr Arg Lys
                645                 650                 655
Ile Ser Val Ile Thr Ile Phe Gly Pro Val Asn Asn Ser Thr Met Lys
            660                 665                 670
Ile Asp His Phe Gln Leu Asp Asn Glu Lys Pro Met Arg Val Val Asp
        675                 680                 685
Asp Glu Asp Leu Val Asp Gln Arg Leu Ile Ser Glu Leu Arg Lys Glu
    690                 695                 700
Tyr Gly Met Thr Tyr Asn Asp Phe Phe Met Val Leu Thr Asp Val Asp
705                 710                 715                 720
Leu Arg Val Lys Gln Tyr Tyr Glu Val Pro Ile Thr Met Lys Ser Val
                725                 730                 735
Phe Asp Leu Ile Asp Thr Phe Gln Ser Arg Ile Lys Asp Met Glu Lys
            740                 745                 750
Gln Lys Lys Glu Gly Ile Val Cys Lys Glu Asp Lys Lys Gln Ser Leu
        755                 760                 765
Glu Asn Phe Leu Ser Arg Phe Arg Trp Arg Arg Arg Leu Leu Val Ile
    770                 775                 780
Ser Ala Pro Asn Asp Glu Asp Trp Ala Tyr Ser Gln Gln Leu Ser Ala
785                 790                 795                 800
Leu Ser Gly Gln Ala Cys Asn Phe Gly Leu Arg His Ile Thr Ile Leu
                805                 810                 815
Lys Leu Leu Gly Val Gly Glu Glu Val Gly Gly Val Leu Glu Leu Phe
            820                 825                 830
Pro Ile Asn Gly Ser Ser Val Val Glu Arg Glu Asp Val Pro Ala His
        835                 840                 845
Leu Val Lys Asp Ile Arg Asn Tyr Phe Gln Val Ser Pro Glu Tyr Phe
    850                 855                 860
Ser Met Leu Leu Val Gly Lys Asp Gly Asn Val Lys Ser Trp Tyr Pro
865                 870                 875                 880
Ser Pro Met Trp Ser Met Val Ile Val Tyr Asp Leu Ile Asp Ser Met
                885                 890                 895
Gln Leu Arg Arg Gln Glu Met Ala Ile Gln Gln Ser Leu Gly Met Arg
            900                 905                 910
Cys Pro Glu Asp Glu Tyr Ala Gly Tyr Gly Tyr His Ser Tyr His Gln
        915                 920                 925
Gly Tyr Gln Asp Gly Tyr Gln Asp Asp Tyr Arg His His Glu Ser Tyr
    930                 935                 940
His His Gly Tyr Pro Tyr
945                 950

<210> 14
<211> 929
<212> PRT
<213> Homo sapiens

<400> 14
Glu Pro His Pro His Ala Thr Ile Arg Gly Ser His Gly Gly Arg Lys
1               5                   10                  15
Val Pro Leu Val Ser Pro Asp Ser Ser Arg Pro Ala Arg Phe Leu Arg
            20                  25                  30
His Thr Gly Arg Ser Arg Gly Ile Glu Arg Ser Thr Leu Glu Glu Pro
        35                  40                  45
Asn Leu Gln Pro Leu Gln Arg Arg Arg Ser Val Pro Val Leu Arg Leu
    50                  55                  60
Ala Arg Pro Thr Glu Pro Pro Ala Arg Ser Asp Ile Asn Gly Ala Ala
65                  70                  75                  80
Val Arg Pro Glu Gln Arg Pro Ala Ala Arg Gly Ser Pro Arg Glu Met
                85                  90                  95
Ile Arg Asp Glu Gly Ser Ser Ala Arg Ser Arg Met Leu Arg Phe Pro
            100                 105                 110
Ser Gly Ser Ser Ser Pro Asn Ile Leu Ala Ser Phe Ala Gly Lys Asn
        115                 120                 125
Arg Val Trp Val Ile Ser Ala Pro His Ala Ser Glu Gly Tyr Tyr Arg
    130                 135                 140
Leu Met Met Ser Leu Leu Lys Asp Asp Val Tyr Cys Glu Leu Ala Glu
145                 150                 155                 160
Arg His Ile Gln Gln Ile Val Leu Phe His Gln Ala Gly Glu Glu Gly
                165                 170                 175
Gly Lys Val Arg Arg Ile Thr Ser Glu Gly Gln Ile Leu Glu Gln Pro
            180                 185                 190
Leu Asp Pro Ser Leu Ile Pro Lys Leu Met Ser Phe Leu Lys Leu Glu
        195                 200                 205
Lys Gly Lys Phe Gly Met Val Leu Leu Lys Lys Thr Leu Gln Val Glu
    210                 215                 220
Glu Arg Tyr Pro Tyr Pro Val Arg Leu Glu Ala Met Tyr Glu Val Ile
225                 230                 235                 240
Asp Gln Gly Pro Ile Arg Arg Ile Glu Lys Ile Arg Gln Lys Gly Phe
                245                 250                 255
Val Gln Lys Cys Lys Ala Ser Gly Val Glu Gly Gln Val Val Ala Glu
            260                 265                 270
Gly Asn Asp Gly Gly Gly Gly Ala Gly Arg Pro Ser Leu Gly Ser Glu
        275                 280                 285
Lys Lys Lys Glu Asp Pro Arg Arg Ala Gln Val Pro Pro Thr Arg Glu
    290                 295                 300
Ser Arg Val Lys Val Leu Arg Lys Leu Ala Ala Thr Ala Pro Ala Leu
305                 310                 315                 320
Pro Gln Pro Pro Ser Thr Pro Arg Ala Thr Thr Leu Pro Pro Ala Pro
                325                 330                 335
Ala Thr Thr Val Thr Arg Ser Thr Ser Arg Ala Val Thr Val Ala Ala
            340                 345                 350
Arg Pro Met Thr Thr Thr Ala Phe Pro Thr Thr Gln Arg Pro Trp Thr
        355                 360                 365
Pro Ser Pro Ser His Arg Pro Pro Thr Thr Thr Glu Val Ile Thr Ala
    370                 375                 380
Arg Arg Pro Ser Val Ser Glu Asn Leu Tyr Pro Pro Ser Arg Lys Asp
385                 390                 395                 400
Gln His Arg Glu Arg Pro Gln Thr Thr Arg Arg Pro Ser Lys Ala Thr
                405                 410                 415
Ser Leu Glu Ser Phe Thr Asn Ala Pro Pro Thr Thr Ile Ser Glu Pro
            420                 425                 430
Ser Thr Arg Ala Ala Gly Pro Gly Arg Phe Arg Asp Asn Arg Met Asp

435                 440                 445
Arg Arg Glu His Gly His Arg Asp Pro Asn Val Val Pro Gly Pro Pro
    450                 455                 460
Lys Pro Ala Lys Glu Lys Pro Pro Lys Lys Lys Ala Gln Asp Lys Ile
465                 470                 475                 480
Leu Ser Asn Glu Tyr Glu Glu Lys Tyr Asp Leu Ser Arg Pro Thr Ala
                485                 490                 495
Ser Gln Leu Glu Asp Glu Leu Gln Val Gly Asn Val Pro Leu Lys Lys
            500                 505                 510
Ala Lys Glu Ser Lys Lys His Glu Lys Leu Glu Lys Pro Glu Lys Glu
        515                 520                 525
Lys Lys Lys Lys Met Lys Asn Glu Asn Ala Asp Lys Leu Leu Lys Ser
    530                 535                 540
Glu Lys Gln Met Lys Lys Ser Glu Lys Lys Ser Lys Gln Glu Lys Glu
545                 550                 555                 560
Lys Ser Lys Lys Lys Lys Gly Gly Lys Thr Glu Gln Asp Gly Tyr Gln
                565                 570                 575
Lys Pro Thr Asn Lys His Phe Thr Gln Ser Pro Lys Lys Ser Val Ala
            580                 585                 590
Asp Leu Leu Gly Ser Phe Glu Gly Lys Arg Arg Leu Leu Leu Ile Thr
        595                 600                 605
Ala Pro Lys Ala Glu Asn Asn Met Tyr Val Gln Gln Arg Asp Glu Tyr
    610                 615                 620
Leu Glu Ser Phe Cys Lys Met Ala Thr Arg Lys Ile Ser Val Ile Thr
625                 630                 635                 640
Ile Phe Gly Pro Val Asn Asn Ser Thr Met Lys Ile Asp His Phe Gln
                645                 650                 655
Leu Asp Asn Glu Lys Pro Met Arg Val Val Asp Asp Glu Asp Leu Val
            660                 665                 670
Asp Gln Arg Leu Ile Ser Glu Leu Arg Lys Glu Tyr Gly Met Thr Tyr
        675                 680                 685
Asn Asp Phe Phe Met Val Leu Thr Asp Val Asp Leu Arg Val Lys Gln
    690                 695                 700
Tyr Tyr Glu Val Pro Ile Thr Met Lys Ser Val Phe Asp Leu Ile Asp
705                 710                 715                 720
Thr Phe Gln Ser Arg Ile Lys Asp Met Glu Lys Gln Lys Lys Glu Gly
                725                 730                 735
Ile Val Cys Lys Glu Asp Lys Lys Gln Ser Leu Glu Asn Phe Leu Ser
            740                 745                 750
Arg Phe Arg Trp Arg Arg Arg Leu Leu Val Ile Ser Ala Pro Asn Asp
        755                 760                 765
Glu Asp Trp Ala Tyr Ser Gln Gln Leu Ser Ala Leu Ser Gly Gln Ala
    770                 775                 780
Cys Asn Phe Gly Leu Arg His Ile Thr Ile Leu Lys Leu Leu Gly Val
785                 790                 795                 800
Gly Glu Glu Val Gly Gly Val Leu Glu Leu Phe Pro Ile Asn Gly Ser
                805                 810                 815
Ser Val Val Glu Arg Glu Asp Val Pro Ala His Leu Val Lys Asp Ile
            820                 825                 830
Arg Asn Tyr Phe Gln Val Ser Pro Glu Tyr Phe Ser Met Leu Leu Val
        835                 840                 845
Gly Lys Asp Gly Asn Val Lys Ser Trp Tyr Pro Ser Pro Met Trp Ser
    850                 855                 860
Met Val Ile Val Tyr Asp Leu Ile Asp Ser Met Gln Leu Arg Arg Gln
865                 870                 875                 880
Glu Met Ala Ile Gln Gln Ser Leu Gly Met Arg Cys Pro Glu Asp Glu
                885                 890                 895

Tyr Ala Gly Tyr Gly Tyr His Ser Tyr His Gln Gly Tyr Gln Asp Gly
            900                 905                 910
Tyr Gln Asp Asp Tyr Arg His His Glu Ser Tyr His His Gly Tyr Pro
        915                 920                 925
Tyr

<210> 15
<211> 19
<212> PRT
<213> Homo sapiens

<400> 15
Tyr Pro Ser Pro Met Trp Ser Met Val Ile Val Tyr Asp Leu Ile Asp
1               5                   10                  15
Ser Met Gln

<210> 16
<211> 20
<212> PRT
<213> Homo sapiens

<400> 16
Tyr Phe Gln Val Ser Pro Glu Tyr Phe Ser Met Leu Leu Val Gly Lys
1               5                   10                  15
Asp Gly Asn Val
            20

## Description

### BACKGROUND OF THE INVENTION

The teachings of all of the references cited herein are incorporated in their entirety herein by reference.

Psoriasis is one of the most common dermatologic diseases, affecting up to 1 to 2 percent of the world's population. It is a chronic inflammatory skin disorder characterized by erythematous, sharply demarcated papules and rounded plaques, covered by silvery micaceous scale. The skin lesions of psoriasis are variably pruritic. Traumatized areas often develop lesions of psoriasis. Additionally, other external factors may exacerbate psoriasis including infections, stress, and medications, e.g. lithium, beta blockers, and anti-malarials.

The most common variety of psoriasis is called plaque type. Patients with plaque-type psoriasis will have stable, slowly growing plaques, which remain basically unchanged for long periods of time. The most common areas for plaque psoriasis to occur are the elbows knees, gluteal cleft, and the scalp. Involvement tends to be symmetrical. Inverse psoriasis affects the intertriginous regions including the axilla, groin, submammary region, and navel, and it also tends to affect the scalp, palms, and soles. The individual lesions are sharply demarcated plaques but may be moist due to their location. Plaque-type psoriasis generally develops slowly and runs an indolent course. It rarely spontaneously remits.

Eruptive psoriasis (guttate psoriasis) is most common in children and young adults. It develops acutely in individuals without psoriasis or in those with chronic plaque psoriasis. Patients present with many small erythematous, scaling papules, frequently after upper respiratory tract infection with beta-hemolytic streptococci. Patients with psoriasis may also develop pustular lesions. These may be localized to the palms and soles or may be generalized and associated with fever, malaise, diarrhea, and arthralgias..

About half of all patients with psoriasis have fingernail involvement, appearing as punctate pitting, nail thickening or subungual hyperkeratosis. About 5 to 10 percent of patients with psoriasis have associated joint complaints, and these are most often found in patients with fingernail involvement. Although some have the coincident occurrence of classic Although some have the coincident occurrence of classic rheumatoid arthritis, many have joint disease (psoriatic arthritis) that falls into one of five type associated with psoriasis: (1) disease limited to a single or a few small joints (70 percent of cases); (2) a seronegative rheumatoid arthritis-like disease; (3) involvement of the distal interphalangeal joints; (4) severe destructive arthritis with the development of "arthritis mutilans"; and (5) disease limited to the spine.

A number of treatments exist for psoriasis but they do not result in satisfactory remission of the disease. Thus there is a need to discover new therapies that more effectively treat the disease.

### DESCRIPTION OF THE INVENTION

The present invention fills this need by providing for a method for treating psoriasis or psoriatic arthritis, which comprises administering to a mammal afflicted with psoriasis or psoriatic arthritis an antagonist to interleukin-17 (also known as Ztgfβ-9). The antagonist to IL-17D can be an antibody, antibody fragment or single-chain antibody that binds to IL-17D, a soluble receptor that binds to IL-17D. Also an antagonist to the IL-17D receptor can be used to treat the disease, such as an antibody, antibody fragment, single-chain antibody or small molecule that binds to the IL-17D receptor. Also an anti-sense nucleotide that binds to the mRNA that encodes IL-17D can be used as an antagonist. A preferred antagonist to IL-17D is the Membrane-Spanning Protein-5 (MSP-5), SEQ ID NOs.: 12 and 13, the mature extracellular portion of the polypeptide being comprised of SEQ ID NO: 14. A preferred embodiment is a soluble receptor SEQ ID NO: 15, corresponding to amino acid residues 879-898 of SEQ ID NO:13, or the soluble receptor SEQ ID NO:16 corresponding to amino acid residues 856-875 of SEQ ID NO:13.

IL-17D is defined and methods for producing it and antibodies to IL-17D are contained in International Patent Application No. PCT/US99/21677, filed September 17,1999, and U.S. Patent Application No. 09/397,846 filed September 17,1999. The polynucleotide and polypeptide of human IL-17D are represented by SEQ ID NOs: 1 - 8, and mouse IL-17D by SEQ ID NOs: 9-11. The MSP-5 sequences are SEQ ID NOs: 12-14, described in International Patent Application No. PCT/US99/05073 and SEQ ID NOs: 15 and 16 are extracellular domains. Another inhibitor would be an anti-idiotypic antibody to MSP-5 that also binds to IL-17D. The present invention also comprises a method for down-regulating IL-17D comprising administering an MSP-5 polypeptide that binds to IL-17D to an individual.

Molecular weights and lengths of polymers determined by imprecise analytical methods (e.g., gel electrophoresis) will be understood to be approximate values. When such a value is expressed as "about" X or "approximately" X, the stated value of X will be understood to be accurate to ±10%.

As used herein, the term "antibodies" includes polyclonal antibodies, affinity-purified polyclonal antibodies, monoclonal antibodies, and antigen-binding fragments, such as F(ab')₂ and Fab proteolytic fragments. Genetically engineered intact antibodies or fragments, such as chimeric antibodies, Fv fragments, single chain antibodies and the like, as well as synthetic antigen-binding peptides and polypeptides, are also included. Non-human antibodies may be humanized by grafting non-human CDRs onto human framework and constant regions, or by incorporating the entire non-human variable domains (optionally "cloaking" them with a human-like surface by replacement of exposed residues, wherein the result is a "veneered" antibody). In some instances, humanized antibodies may retain non-human residues within the human variable region framework domains to enhance proper binding characteristics. Through humanizing antibodies, biological half-life may be increased, and the potential for adverse immune reactions upon administration to humans is reduced. The binding affinity of an antibody can be readily determined by one of ordinary skill in the art, for example, by Scatchard analysis. A variety of assays known to those skilled in the art can be utilized to detect antibodies that bind to protein or peptide. Exemplary assays are described in detail in Antibodies: A Laboratory Manual, Harlow and Lane (Eds.) (Cold Spring Harbor Laboratory Press, 1988). Representative examples of such assays include: concurrent immunoelectrophoresis, radioimmunoassay, radioimmunoprecipitation, enzyme-linked immunosorbent assay (ELISA), dot blot or Western blot assay, inhibition or competition assay, and sandwich assay.

### Use of Antagonist to IL-17D to Treat Psoriasis

As indicated in the discussion above and the examples below, IL-17D is involved in the pathology of psoriasis. The present invention is in particular a method for treating psoriasis by administering antagonists to IL-17D. The antagonists to IL-17D can either be a soluble receptor such as SEQ ID NOs:15 and 16 that binds to IL-17D or antibodies, single chain antibodies or fragments of antibodies that bind to either IL-17D or the IL-17D receptor (SEQ ID NOs: 13 and 14).

### Administration of Antagonists to IL-17D

The quantities of antagonists to IL-17D necessary for effective therapy will depend upon many different factors, including means of administration, target site, physiological state of the patient, and other medications administered. Thus, treatment dosages should be titrated to optimize safety and efficacy. Typically, dosages used in *vitro* may provide useful guidance in the amounts useful for *in vivo* administration of these reagents. Animal testing of effective doses for treatment of particular disorders will provide further predictive indication of human dosage. Methods for administration include oral, intravenous, peritoneal, intramuscular, transdermal or administration into the lung or trachea in spray form by means or a nebulizer or atomizer. Pharmaceutically acceptable carriers will include water, saline, buffers to name just a few. Dosage ranges would ordinarily be expected from 1µg to 1000µg per kilogram of body weight per day. A dosage of MSP-5 or an antibody that binds to IL-17D would be about 25 mg given twice weekly. For subcutaneous or intravenous administration of the antagonist to IL-17D, the antibody or MSP-5 can be in phosphate buffered saline. Also in skin diseases such as psoriasis, the antagonist to IL-17D can be administered via an ointment or transdermal patch. The doses by may be higher or lower as can be determined by a medical doctor with ordinary skill in the art. For a complete discussion of drug formulations and dosage ranges *see* Remington's Pharmaceutical Sciences, 18th Ed., (Mack Publishing Co., Easton, Penn., 1996), and Goodman and Gilman's: The Pharmacological Bases of Therapeutics, 9th Ed. (Pergamon Press 1996).

The invention is further illustrated by the following non-limiting examples.

### Example 1

### In situ Hybridization of IL-17D

Spatial distribution of *IL-17D* mRNA in normal and diseased skin tissues were studied using *in situ* hybridization analysis. In the skin sample analyzed, only psoriasis samples have keratinocyte signal. The hybridization results indicated that IL-17D was highly expressed in the skin of psoriasis patients, and to a lesser degree in the skin of patients with lichen planus.

### Example 2

### MSP-5 Binds to IL-17D

Two assays were used to determine that MSP-5 binds to IL-17D. The first was a secretion trap technique, which revealed that MSP-5 expressed from the human keratinocyte cell line, HaCAT bound to IL-17Dβ-9. This was confirmed by transfecting BHK cells with the *MSP-5* cDNA and showing that these transfected cells bound to iodinated IL-17Dβ-9

### CLAUSES

Clause 1. A method for treating a mammal afflicted with psoriasis comprised of administering an antagonist to a polypeptide selected from the group consisting of SEQ ID NOs : 2, 3, 4, 5, 7, 8, 10 and 11.
Clause 2. The method of clause 1 wherein the antagonist is an antibody, antibody fragment or a single-chain antibody.
Clause 3. The method of clause 1 wherein the antagonist is comprised of a polypeptide selected from the group consisting of SEQ ID NOs: 13, 14, 15 and 16 or a subsequence thereof.
Clause 4. A method for down-regulating a polypeptide selected from the group consisting of SEQ ID NOs: 2, 3, 4, 5, 7, 8, 10 and 11 comprising administering a therapeutically effective dose of SEQ ID NOs: 15 or 16.
Clause 5. The use of an antagonist to a polypeptide selected from the group consisting of SEQ ID NOs: 2, 3, 4, 5, 7, 8, 10 and 11 for the production of a medicament for the treatment of psoriasis or psoriatic arthritis.
Clause 6. The use of clause 5 wherein the antagonist is an antibody, antibody fragment or single-chain antibody that binds to said polypeptide.
Clause 7. The use of clause 5 wherein the antagonist is a polypeptide selected from the group consisting of SEQ ID NOs: 13, 14, 15 and 16 or a subsequence thereof.
Clause 8. The use of clause 5 wherein the antagonist is an antibody, antibody fragment or single-chain antibody that binds to a polypeptide selected from the group consisting of SEQ ID NOs: 13, 14, 15 and 16.

## Claims

1. Use of an antagonist to a polypeptide selected from the group consisting of SEQ ID. NOs: 2, 3, 5, 7, and 8 in the manufacture of a medicament for the treatment of psoriatic arthritis, wherein said antagonist is antibody, antibody fragment, or single-chain antibody.

2. Use of an antagonist to a polypeptide selected from the group consisting of SEQ ID. NOs: 2, 3, 5, 7, and 8 in the manufacture of a medicament for the treatment of psoriatic arthritis, wherein said antagonist is a polypeptide selected from the group consisting of SEQ ID. NOs: 13, 14, 15, and 16 or a subsequence thereof.

3. Use of an antagonist to a polypeptide selected from the group consisting of SEQ ID. NOs: 2, 3, 5, 7, and 8 in the manufacture of a medicament for the treatment of psoriatic arthritis, wherein said antagonist is an antisense nucleotide that binds to mRNA that encodes said polypeptide.

4. An antagonist to a polypeptide selected from the group consisting of SEQ ID. NOs: 2, 3, 5, 7, and 8 for use in treating psoriatic arthritis, wherein said antagonist is antibody, antibody fragment or single chain antibody that binds to said polypeptide.

5. An antagonist to a polypeptide selected from the group consisting of SEQ ID. NOs: 2, 3, 5, 7, and 8 for use in treating psoriatic arthritis, wherein said antagonist is a polypeptide selected from the group consisting of SEQ ID. NOs: 13, 14, 15 and 16 or a subsequence thereof.

6. An antagonist to a polypeptide selected from the group consisting of SEQ ID. NOs: 2, 3, 5, 7, and 8 for use in treating psoriatic arthritis, wherein said antagonist is an anti-sense nucleotide that binds to mRNA that encodes said polypeptide.
